# EUROPEAN PATENT SPECIFICATION

(11) **EP 1 217 990 B1**
(45) Date of publication and mention of the grant of the patent: **28.01.2004**
(21) Application number: 00967824.4
(22) Date of filing: 29.09.2000
(51) Int. Cl.: A61K 9/127

(54) **CATIONIC DOSPER VIROSOMES**
KATIONISCHE DOSPER VIROSOMEN
VIROSOMES DOSPER CATIONIQUES

(30) Priority: 08.10.1999 US 414872
(43) Date of publication of application: 03.07.2002
(73) Proprietor: NIKA HEALTH PRODUCTS LIMITED, FL-9490 Vaduz (LI)
(72) Inventor: WÄLTI, Ernst, Rudolf, CH-3053 Münchenbuchsee (CH); GLÜCK, Reinhard, CH-3028 Spiegel bei Bern (CH); KLEIN, Peter, CH-4438 Langenbruck (CH)
(74) Representative: Büchel, Kurt F.
(86) International application number: PCT/EP2000/009540
(87) International publication number: WO 2001/026628

(56) References cited:
- EP-A- 0 819 758
- WO-A-97/00241
- WO-A-97/41834
- BUCHBERGER ET AL: "DOSPER liposomal transfection reagent: a reagent with unique transfection properties" BIOCHEMICA,MANNHEIM,DE, no. 2, 1996, pages 7-10, XP002088278 ISSN: 0946-1310

## Description

### FIELD OF INVENTION

The present invention is in the fields of biochemistry, gene biotechnology and gene therapy and relates to novel virosomes, i.e., positively charged liposomal vesicles containing viral glycoproteins in the membrane, for highly efficient transfer of desired material, particularly genetic material, to target locations, and useful applications thereof. The present cationic virosomes are particularly suitable for the specific and unspecific, non-infectious delivery of negatively charged compounds, preferably genes, to target cells in vitro and in vivo.

### BACKGROUND OF THE INVENTION

WO 92/13525 reports that virosomes made of phospholipid bilayer membranes which are targeted with viral spike proteins from influenza virus and with cell-specific markers such as, e.g., monoclonal antibodies, very efficiently fuse with model membranes and animal cells due to a virus-like penetration mechanism by way of receptor-mediated endocytosis. While these virosomes are successfully applied to deliver chemical substances and desired drugs to target locations, they suffer from certain disadvantages with respect to stable incorporation and transfer of charged molecules such as, for instance, negatively charged nucleic acids.

WO97/41834, reports cationic virosomes for efficient cell-specific delivery of genetic material to target cells in vitro and in vivo. It further reports a way of manufacture of such virosomes as well as methods for the application of these virosomes.

### SUMMARY OF THE INVENTION

The present invention is a further development of the invention reported in WO97/41834 and relates to a novel cationic virosome which due to its specific membrane composition may very efficiently be loaded with any desired negatively charged material, preferably with genetic material comprising long and short chain DNA or RNA, oligodeoxynucleotides, ribonucleotides, peptide nucleic acids (PNA), ribozymes (RNA molecules with enzymatic activities), genes, plasmids and vectors. Unexpectedly, it has been found that when using DOSPER as a cationic lipid in combination with other lipids, high performance virosomes can be produced that even exceed up to ten times the already high transfection efficiency of the DOTAP virosomes of WO97/41834. This surprising effect is only achieved, however, when DOSPER is used as a cationic lipid and when DOSPER is combined with other lipids at a ratio that is different from the known ratio of the cationic lipids (DOTAP) to the other lipids explicitly disclosed and claimed in WO97/41834.

### BRIEF DESCRIPTION OF THE DRAWINGS

- Fig. 1:: Influence of length of time of transfection on the amount of uptaken DNA-plasmid. 5x10⁵ cells of human, transformed primary embryonal kidney cell line 293 T in 0.5 ml medium were transfected with DOSPER-virosomes and DOSPER-liposomes containing ³H-labeled plasmid pGreen Lantern.
- Fig. 2:: Influence of length of time of transfection on the amount of uptaken DNA-plasmid. 5x10⁵ cells of monkey fibroblast cell line COS-1 were transfected with DOSPER-virosomes and DOSPER-lipsomes containing ³H-labeled plasmid pGreen Lantern.
- Fig. 3:: Influence of length of time of transfection on the amount of uptaken DNA-plasmid. 5x10⁵ cells of human epithelioid cervix carcinoma cell line HeLa in 0.5 ml medium were transfected with DOSPER-virosomes and DOSPER-liposomes containing ³H-labeled plasmid pGreen Lantern.
- Fig. 4:: Influence of length of time of transfection on the amount of uptaken DNA-plasmid. 5x10⁵ cells of mouse fibroblast cell line NIH3T3 were transfected with DOSPER-virosomes and DOSPER-liposomes containing ³H-labeled plasmid pGreen Lantern.
- Fig. 5:: Influence of length of time of transfection on the amount of uptaken DNA-plasmid. 5x10⁵ cells of human chronic myelogenous leukemia cell line K562 were transfected with DOSPER-virosomes and DOSPER-liposomes containing ³H-labeled plasmid pGreen Lantern.
- Fig. 6:: Influence of length of time of transfection on the amount of uptaken DNA-plasmid. 5x10⁵ cells of mouse myeloma cell line P3 were transfected with DOSPER-virosomes and DOSPER-liposomes containing ³H-labeled plasmid pGreen Lantern.
- Fig. 7:: Influence of length of time of transfection on the amount of uptaken DNA-plasmid. 5x10⁵ cells of cell lines COS-1, 293T, K562 and P3X63Ag8 were transfected with DOSPER-virosomes containing ³H-labeled plasmid pcDNA3.1/His B/lacZ.
- Fig. 8:: Influence of length of time of transfection on the amount of uptaken DNA-plasmid. 5x10⁵ cells of human, transformed primary embryonal kidney cell line 293 T in 0.5 ml of medium were transfected with DOSPER-virosomes and DOTAP-virosomes containing ³H-labeled plasmid pcDNA3. 1/His B/lacZ his (0.4 µg DNA; 24'000 dpm = 100%).
- Fig. 9:: Influence of length of time of transfection on the amount of uptaken DNA-plasmid. 5x10⁵ cells of human chronic myelogenous leukemia cell line K562 in 0.5 ml of medium were transfected with DOSPER-virosomes and DOTAP-virosomes containing ³H-labeled plasmid pcDNA3.1/His B/lacZ his (0.4 µg DNA; 24'000 dpm = 100%).
- Fig. 10:: Influence of length of time of transfection on the amount of uptaken DNA-plasmid. 5x10⁵ cells of the hybrid, non-secreting, mouse cell line Sp2 in 0.5 ml of medium were transfected with DOSPER-virosomes and DOTAP-virosomes containing ³H-labeled plasmid pcDNA3.1/His B/lacZ his (0.4 µg DNA; 24'000 dpm = 100%).
- Fig. 11:: Influence of length of time of transfection on the amount of uptaken DNA-plasmid. 5x10⁵ cells of human epithelioid carcinoma cell line HeLa were transfected with DOSPER-virosomes and DOTAP-virosomes containing ³H-labeled plasmid pGreen Lantern.
- Fig.: Expression of β-gatactosidase in NIH3T3 cells that have been transfected with DOSPER virosomes or DOSPER liposomes, respectively; mean values of three experiments A, B and C.
- Fig. 13:: Expression of β-galactosidase in NIH3T3 and 293T cells that have been transfected with DOSPER virosomes (experiment A) using plasmid pcDNA3.1/His B/lacZ in different concentrations and supplied through different numbers of virosomes; values in ng/mg protein after 4 days.

### DETAILED DESCRIPTION OF PREFERRED EMBODIMENTS

The virosomes of the present invention are lipid vesicles that typically comprise a vesicle membrane having a positive net charge due to the presence of cationic lipids. They further comprise fusogenic peptides of viral origin, predominantly glycopeptides of viral envelopes such as influenza virus hemagglutinin, which peptides cause the virosomes to be incorporated by the target cells via a receptor-mediated penetration mechanism. They may optionally further be equipped with cell-specific markers for preferred attachment to the desired target cells in vivo.

The presence of phosphatidylethanolamine (PE) in the membrane is preferred for anchoring such a cell-specific marker to the virosome by means of a bifunctional crosslinker. Where cell-specific targeting of the virosomes is not required (e.g., for application in defined cell cultures in vitro) or where the cell-specific marker is attached to the membrane other than by way of a bifunctional crosslinker, PE may optionally be absent from the virosome membrane.

In one embodiment that is preferred for in vivo applications of the virosomes, the present invention also relates to the irreversible covalent linkage of cell-specific markers to the cationic virosomes including but not being limited to monoclonal antibodies, antibody fragments such as F(ab')2 and Fab' fragments, cytokines, and/or growth factors, useful for a selective detection and binding of target cells. They are linked to the vesicle membrane such that they extend to the exterior and exert essentially full functional activity with respect to receptor detection and binding. A preferred method for making virosomes comprising the site-oriented cross-linkage of a cell-specific marker is disclosed in Example 1 of WO97/41834 for DOTAP virosomes. According to that method, the cell-specific markers are coupled to preformed phosphatidylethanolamine-crosslinker molecules such as, for example, N-[4-(p-maleimido-phenylbutyryl]-phosphatidylethanolamine (MPB.PE) in the presence of a detergent.

In order to achieve the best possible results it is advantageous to carefully isolate and purify the viral glycoproteins before reconstitution in order to avoid inactivation by either proteolytic digestion or reduction of intramolecular S-S bonds. Accordingly, it is preferred that the conjugated markers, e.g., marker-MPB.PE, be separated from unconjugated phosphatidylethanolamine-crosslinker molecules (e.g., MPB.PE) by affinity chromatography with an activated agarose matrix, preferably with reduced Thiopropyl Sepharose 6B. Aliquots of the purified conjugated phosphatidylethanolamine-crosslinker-marker molecule complexes are then added to the detergent solution containing the mixture of dissolved membrane lipids, fusion peptides and other desired ingredients, before the cationic virosomes are formed thereof.
It is advantageous to carry out the coupling procedure of the bifunctional crosslinker with the phospholipid and the cell-specific marker in a separate process prior to the preparation of the virosomes. This procedure allows to better control and optimize the surface density of the virosome membranes, particularly with respect to the number of cell-specific markers linked thereto. The improved control of the concentration of protein molecules embedded in or linked to the membrane is important in as much as an unbalanced ratio of fusion peptides (e.g., hemagglutinin) and cell-specific markers (e.g., antibody Fab' fragments) on the virosome membrane may reduce or even destroy their selective properties and - at the extreme - may result in clotting and precipitation of the vesicles.

The advantage of using antibody fragments F(ab')2 and Fab' instead of whole antibody molecules as cell-specific markers has been broadly discussed in WO 97/41834.The present cell-specific virosomes exert a selectivity for various cell types owing to their cell-specific markers on the membrane and, simultaneously, a high capability for cell penetration by endocytosis owing to the viral fusogenic peptide, e.g., hemagglutinin. Virosomes with Fab' fragments that recognize tumor-associated antigens such as TAG72, CEA, 17-1A, CA 19-9 or leukemia-associated antigens such as CD10 (CALLA = Common Acute Lymphocytic Leukemia Antigen) and CD20 bind selectively, i.e. more preferably, to tumor or leukemia cells carrying the mentioned antigens on their cell surface.

The novel vesicles or virosomes are particularly useful to transfer any desired negatively charged material, preferably genetic material, to target locations, in particular to animal and human cells and tissues in vitro and in vivo. It is emphasized that the novel virosomes are not only able to penetrate proliferating, i.e., replicating cells but also non-proliferating, i.e., resting cells, which feature makes them widely applicable in the fields of biosciences, pharmacology and medicine, both as a research and/or diagnostic tool and as a medicament. Using the present virosomes as shuttles for the delivery of cytotoxic agents or nucleic acid material makes them suitable for applications in various pathological conditions such as, for example, viral infections, cancers, tumors, leukemias, or other diseases including diseases that are due to genetic defects and that may be susceptible to gene therapy methods using the present virosomes.

The virosomes of the present invention may be applied *in vitro* or *in vivo.* For instance, purging of bone marrow is used as a component in the treatment of several neoplasms, including acute and chronic leukemias. At present, marrow is cleansed of leukemic cells by a variety of agents such as immunologic reagents and chemotherapeutic drugs. Virosome-encapsulated ODN targeted against one oncogene that confers a growth advantage to leukemic cells will prove therapeutically useful and, most important, more selective than conventional chemotherapeutic agents in eliminating leukemic cells while sparing normal progenitor cells.

For the use as a medicament, the present virosomes may be part of a pharmaceutical composition which further comprises usual additives and pharmaceutically suitable carriers. It is preferred that the pharmaceutical composition is prepared as an injection solution, but other forms of preparation, e.g., emulsions, cremes, gels, ointments, for topical or systemic administration may be advantageous for some applications.

Therefore, it is also an objective of the present invention to use the present virosomes for the manufacture of a pharmaceutical composition suitable for the prophylactic and/or therapeutic treatment of animal or human individuals who may benefit from such treatment. It is another objective of the present invention to use the present virosomes for the manufacture of a diagnostic kit for in vitro and in vivo applications.

The present vesicles are preferably obtained by a process analogous to any one of the processes for making DOTAP virosomes disclosed in Examples 1 to 3 and 6 of WO97/41834, except that DOTAP is replaced by DOSPER and that the DOSPER concentration in the final virosome membrane is properly adjusted as hereinbelow defined and, in particular, does not exceed 30 % by weight of the total lipid content of the virosome. Basically, the method of preparation of the present virosomes comprises the following steps:
a) preparing a buffer solution that comprises a non-ionic detergent and that further comprises DOSPER and other lipids and at least one active fusogenic peptide that is a non-Sendai viral hemagglutinin that causes the vesicles to be internalized by target cells through receptor-mediated phagocytosis or endocytosis;
b) adjusting the lipid concentrations to - based on total membrane lipids - 5 to 30 % by weight of DOSPER and to a balance of 95 to 70 % by weight of said other lipids comprising phosphatidylcholine (PC) or a derivative thereof and optionally phosphatidylethanolamine (PE) and/or cationic lipids other than DOSPER; and
(c) removing the detergent by dialysis or by treating the solution with microcarrier beads, resulting in the formation of said positively charged lipid bilayer vesicles; and preferably
(d) incorporating into the vesicles a quantity of a desired drug or substance for delivery to target cells, said desired drug or substance preferably being negatively charged and particularly being selected from the group consisting of a dye, a tracer substance, a cosmetic agent, a pharmaceutically or biologically active substance, and a nucleic acid material.

In another embodiment of the invention preferred for *in vivo* applications of the virosomes, a suitable bifunctional crosslinker is applied to link a cell-specific marker irreversibly to the vesicle membrane. The cell-specific marker, which is directed to a cell-receptor responsible for the selective binding of the virosome to the cell, is bound to the crosslinker in such a manner that it is still fully biologically active. It is preferred that the crosslinker be employed in the form of a preformed molecule-complex wherein the crosslinker is covalently bound to phosphatidylethanolamine and a cell-specific marker, as defined in the claims.

The term "fusogenic peptide" refers to peptides or proteins capable of inducing and/or promoting a fusion reaction between the virosome membrane and a lipid membrane of the target cell. In most embodiments, it refers to viral spike glycoproteins containing the fusion peptide, particularly to the complete hemagglutinin trimer of viral surface spikes, a monomer thereof, or one or both cleaved subunits, the glycopeptides HA1 and HA2, containing the functional fusion peptide. In another embodiment of the present invention the term refers to the pure fusion peptide itself, either isolated from natural sources or synthetically produced. In a particularly preferred embodiment of the present invention, these polypeptides containing the fusion peptide refer to influenza virus hemagglutinins, e.g. the one of the A-H1N1 subtype.

Instead of or in addition to influenza virus hemagglutinin, the hemagglutinins from other viruses may also be suitably used as fusion peptides (proteins) for the instant virosomes as long as they exert substantially the same pH-mediated cell penetration mechanism as the influenza hemagglutinin. Candidate hemagglutinins include, for example, rhabdovirus, parainfluenza virus type III, Semliki Forest virus and togavirus, as disclosed in WO97/41834.

The term "crosslinker" refers to an organic heterobifunctional molecule capable of linking to the surface of vesicles prepared according to this invention and capable of binding polypeptides. In a preferred embodiment of the present invention, this molecule contains a N-hydroxysuccinimide group for coupling to the amino group of phosphatidylethanolamine and a maleimide group for conjugation of a cell-specific marker such as, for example a monoclonal antibody fragment. Suitable crosslinkers comprise succinimidyl 4-(N-maleimidomethyl)-cyclohexane-1-carboxylate, m-maleimidobenzoyl-N-hydroxysuccinimide ester, m-maleimidobenzoyl-N-hydroxysulfosuccinimide ester, succinimidyl 4(p-maleimidophenyl)-butyrate, sulfosuccinimidyl 4(p-maleimidophenyl)butyrate; alternatively, the crosslinker may be a molecule that contains a N-hydroxysuccinimide group and a photoreactive azido group for coupling to markers, e.g., cytokines, such as N-hydroxysuccinimidylsuberate (NHS-SA), N-hydroxysuccinimidyl-4-azidobenzoate (HASAB), N-succinimidyl-6-(4'-azido-2'-nitrophenylamino)hexanoate (SANPAH), N-sulfosuccinimidyl-6-(4'-azido-2 -nitrophenylamino)hexanoate. It is preferred that the crosslinker be used in the form of a preformed molecule complex of lipid plus crosslinker plus cell-specific marker.

The term "cell-specific protein" or "cell-specific marker" refers to a protein capable of linking to the crosslinker or crosslinker-lipid complex, respectively, and further being capable of binding to the receptor of target cells. In a preferred embodiment of the present invention, this molecule refers to a monoclonal antibody, an antibody fragment, a cytokine or a growth factor. The cell-specific marker provides for selective detection and binding of target cells and thus improves the action of the fusogenic peptide concomitantly present in the virosomal membrane. The preferred antibody fragments comprise the F(ab')2 and Fab' fragments, while the cell-specific markers further comprise interleukins and other cytokines, particularly the ones listed in Table 1 below.

**Table 1**

| Cytokines (international abbreviations) | | | |
|---|---|---|---|
| BDNF | IFNα | MIP-1α | PDGF |
| CNTF | IFNβ | MIP-1β | PF-4 |
| EGF | IFNγ | MIP-2 | RANTES |
| Epo | IL-1 to IL-15 | NGF | SCF |
| FGF | LIF | NT-3 | TGFα |
| G-CSF | LT (TNFβ) | NT-4 | TGFβ |
| GM-CSF | MCP-1 to MCP-3 | OSM | TNFα |
| 1-309/TCA-3 | M-CSF | PBP | Tpo |
| γIP-10 | MIF | PBSF | jVEGF |

The term "cationic lipid" as used herein refers to an organic molecule that contains a cationic component and a nonpolar tail, a so-called head-to-tail amphiphile, such as N-[(1,2,3-dioleoyloxy)propyl]-N,N,N-trimethylammonium chloride (DOTMA) (Felgner et al.; Proc Natl Acad USA 84:7413-7417, 1987), N-[1,2,3-dioleoyloxy)-propyl]-N,N,N-trimethylammoniummethylsulfate (DOTAP); or N-t-butyl-N'-tetradecyl-3-tetradecylaminopropionamidine (Ruysschaert et al.; Biochem. Biophys. Res. Commun. 203:1622-1628, 1994). Unless explicitly mentioned otherwise, the term also includes the below defined polycationic lipids.

The term "polycationic lipid" refers to an organic molecule that contains a polycationic component and a nonpolar tail such as the lipospermine: 1,3-dipalmitoyl-2-phosphatidylethanolamido-spermine (DPPES) and dioctadecylamidoglycyl spermine (DOGS) (Behr et al. ; Proc. Natl. Acad. USA 86:6982-6986, 1989); 2,3-dioleoyloxy-N-[2(sperminecarboxamido) ethyl]-N,N-dimethyl-1-propaneaminium trifluoroacetate (DOSPA); 1,3-dioleoyloxy-2-(6-carboxy-spermyl)-propylamide (DOSPER); N,N,N',N'-tetramethy(-N,N'-bis(2-hydroxyethyl)-2,3-dioleoyloxy-1,4-butanediammonium iodide (THDOB).

It is essential that all virosomes of the present invention contain DOSPER and one or more other natural or synthetic lipids that are positively charged or neutral. By "positively charged" is meant that the lipids have a positive net charge at physiological pH. Thus, "positively charged" lipids, membranes or virosomes shall mean lipids, membranes or virosomes that have a positive net charge at physiological pH. The "neutral lipids" do not exhibit any net charge at physiological pH and comprise lipids such as cholesterol or derivatives thereof, and/or phospholipids of the zwitterionic-type, which have positively and negatively charged areas within the molecule which stoichiometrically offset each other's charges.

The term "negatively charged material " as used herein comprises any desired drug or substance that bears a negative net charge at physiological pH.

The term "desired drug or substance" as used herein is understood to comprise any chemical substance, compound or mixture of compounds that is applicable for cosmetic, diagnostic, pharmaceutical, medical or any other scientific purposes. Such substances comprise compounds that can be analytically monitored in vitro or in vivo such as dyes, fluorescent dyes, radioactively or otherwise labeled compounds, tracer or marker substances; they also comprise, however, protein or non-protein drugs, or any other pharmaceutically or biologically active substances, particularly conventional anti-viral or anti-cancer agents, but also nucleic acid compounds as herein defined. Since the present virosomes are positively charged at physiological pH it is preferred that they are loaded with either neutral or negatively charged desired drugs or substances, because they allow for the most efficient and stable incorporation into the virosomes.

The terms "nucleic acid" or "genetic material" as used herein comprise short chain DNA or RNA, deoxyribonucleotides, oligodeoxyribonucleotides, oligodeoxyribonucleotide selenoates, oligodeoxyribonucleotide phosphorothioates (OPTs), oligodeoxyribonucleotide phosphoramidates, oligodeoxyribonucleotide methylphosphonates, peptide nucleic acids (PNAs), ribonucleotides, oligoribonucleotides, oligoribonucleotide phosphorothioates, 2'-OMe-oligoribonucleotide phosphates, 2'-OMe-oligoribonucleotide phosphorothioates, ribozymes (RNA molecules with enzymatic activities), genes, plasmids and vectors (cloning vehicles).

The term "virosomes" as used herein refers - in its simplest form - to liposomal vesicles with a bilayer membrane comprising cationic lipids and an internal - preferably aqueous - space, wherein the membrane further contains viral proteins, in particular viral glycoproteins. In the preferred embodiment, the viral proteins comprise at least one fusogenic peptide or protein having full biological fusion activity, particularly the spike glycoprotein hemagglutinin and/or neuraminidase of influenza A (e.g., A/Singapore) virus. It shall be understood that the viral proteins also encompass synthetically produced amino acid sequences corresponding to or equal to the fusion peptide of influenza virus as herein described. The membrane lipids comprise the cationic lipids defined above but may optionally further comprise other natural and/or synthetic lipids, preferably phospholipids such as phosphatidylcholine (PC) and phosphatidylethanolamine (PE).

Although the cationic virosomes of the present invention may in many cases - notably for in vitro cell culture experiments - successfully be applied without cell-specific markers on the membrane, it is particularly preferred for in vivo applications that they further comprise at least one cell-specific marker on the membrane as hereinbefore defined. The mean diameter of the vesicles is in the range of 120 - 180 nm, as determined by electron microscopy and dynamic light scattering.

By using the cationic virosomes of the present invention as carriers for desired drugs or substances including genetic material undesired side effects due to toxicity can be prevented or at least considerably decreased. This beneficial effect is achieved because the present cationic virosomes have - compared to liposomes or virosomes known hitherto - a far higher activity and efficiency of for the transfer of entrapped material, particularly negatively charged material such as antisense oligonucleotides, into target cells and - even more important - for the expression of the transfected nucleic acid material by the target cell.
Accordingly, it is an object of the present invention to provide for the DOSPER virosomes defined in claim 1 and variations thereof defined as further embodiments in the subsequent claims 2 to 11. It is also an object of the invention to provide for a compositon containing the novel virosomes along with a suitable carrier for various applications as defined for instance in claim 12.
It is another object of the present invention to provide for a process of making the novel virosomes as defined in claim 13 with the variations defined in claims 14 to 24.
It is yet another object of the present invention to teach and claim more specific methods of using the present virosomes, as defined in claims 25 to 29.

In order that the invention described herein may be more fully understood, the following examples are set forth. The examples are for illustrative purposes only and are not to be construed as limiting this invention in any respect.

### Example 1: Preparation of DOSPER virosomes with fully fusion-active viral hemagglutinin trimers from influenza virus

Hemagglutinin (HA) from the A/Singapore/6/86 strain of influenza virus was isolated as described by Skehel and Schild (1971), Proc. Natl. Acad. Sci. USA 79:968-972. In short, virus was grown in the allantoic cavity of hen eggs, and was purified twice by ultracentrifugation in a sucrose gradient. Purified virus was stabilized in a buffer containing 7.9 mg/ml NaCl, 4.4 mg/ml trisodiumcitrate-2H2O, 2.1 mg/ml 2-morpholinoethane sulfonic acid, and 1.2 mg/ml N-hydroxyethyl-piperazine-N'-2-ethane sulfonic acid pH 7.3. 53 ml of the virus suspension containing 345 µg HA per ml were pelletted by ultracentrifugation at 100,000 x g for 10 minutes. 7.7 ml of a buffered detergent solution containing 145 mM NaCl, 2.5 mM HEPES and 54 mg/ml of the non-ionic detergent octaethyleneglycol monododecylether (OEG = C12E8), pH 7.4, were added to the influenza virus pellet. The pellet was completely dissolved by using ultrasonication for 2 minutes at room temperature. The solution was subjected to ultracentrifugation at 100,000 x g for 1 hour. The obtained supernatant contained the solubilized HA trimer (1.635 mg HA/ml) and trace amounts of neuraminidase. 0.5 - 2 mg of DOSPER and 5.5 - 4 mg (making up the balance of 6 mg total lipid) of dioleoylphosphatidylcholine (DOPC) were added to 3.7 ml of supernatant (6 mg HA) and dissolved. The solution was sterilized by passage through a 0.2 µm filter and then transferred to a glass container containing 1.15 g of sterile microcarrier beads, preferably Biobeads SM-2. The container was shaken for 1 hour by using a shaker REAX2 from Heidolph (Kelheim, Germany). When necessary, this procedure was repeated up to three times with 0.58 mg of Biobeads. After these procedures a slightly transparent solution of DOSPER virosomes was obtained.

Best results were obtained with virosome preparations that contained a lipid ratio of 1.5 mg DOSPER (25 % by weight of total lipids) per 4.5 mg DOPC. Higher DOSPER concentrations, e.g. above 30 - 35% by weight, did not result in proper virosome formation upon detergent removal.

Partial replacement of DOPC by PE (phosphatidylethanolamine) and/or other cationic lipids such as DOTAP or DOSPA is possible without significant loss of transfection activity.

Incorporation of phosphorothioate oligodeoxyribonucleotides into DOSPER virosomes:
The antisense and sense oligodeoxyribonucleotide phosphorothioates (OPTs) of the L-myc gene were used as an example for the demonstration of the high efficiency of cationic virosomes in transfection. 5'-FITC-OPTs were synthesized via phosphoramidite chemistry (Microsynth GmbH, Balgach, Switzerland). The pentadecamer (5'-FITC-GTAGTCCATGTCCGC-3') and the pentadecamer (5'-FITC-GCGGACATGGACTAC-3') were used as the antisense OPT and sense OPT, respectively. A mixed sequence control (msc) OPT consisting of the same length of nucleotides as antisense and sense OPTs was synthesized.

1 ml of DOSPER virosomes was added to each of
a) 2 mg of antisense FITC-OPT (1.3 µmol)
b) 3.4 mg sense FITC-OPT (1.3 µmol) and
c) 3.1 mg msc FITC-OPT (1.3 µmol).

The FITC-OPTs were dissolved and the solutions were then treated by sonication for 2 minutes at 26°C. Non-encapsulated FITC-OPTs were separated from the virosomes by gel filtration on a High Load Superdex 200 column (Pharmacia, Sweden). The column was equilibrated with sterile PBS. The void volume fractions containing the DOSPER virosomes with encapsulated FITC-OPT were eluted with PBS and collected. Virosome-entrapped FITC-OPT concentrations were determined fluorometrically after the virosomes were fully dissolved in 0.1 M NaOH containing 0.1 % (v/v) Triton X-100. For calibration of the fluorescence scale the fluorescence of empty DOSPER-virosomes that were dissolved in the above detergent solution was set to zero.

Coupling of Fab'-fragments to virosomes by means of preformed phosphatidylethanolamine-bifunctional crosslinker molecule complexes 3 mg of freshly reduced Fab' from murine monoclonal anti-CD10-(AntiCALLA) antibody, dissolved in 2.8 ml of a citric acid buffer solution (100 mM NaCl, 40 mM citric acid, 35 mM Na2HPO4.2H2O, 2 mM EDTA, pH 5.5) were added to a solution of 0.524 mg of N-[4-(p-maleimido-phenyl)butyryl] phosphatidylethanolamine (MPB.PE) in 215 µl of citric acid buffer containing 0.5% of n-octyl-oligo-oxyethylene. The mixture was then incubated under nitrogen for 16 h at 4°C with gentle stirring. After incubation the non-coupled MPB.PE was removed by a batch of 400 µl of freshly reduced wet Thiopropyl Sepharose 6B (Pharmacia, Sweden). The mixture was incubated for 4 h at room temperature. The Thiopropyl Sepharose 6B was removed by centrifugation and the resulting solution neutralized to pH 7.0. The neutralized solution was supplemented with OEG (54 mg/ml).

The solutions prepared as described above were added to the solutions for the preparation of DOSPER virosomes. The Fab'-MPB.PE molecules are inserted into the lipid bilayer during the formation of virosomes.

Micrographs of DOSPER virosomes confirm the preferred unilamellar structure of the vesicles with an average diameter of approximately 120 to 180 nm as determined by laser light scattering. The HA protein spikes of the influenza virus are clearly visible.

The full fuogenic activity of the present DOSPER virosomes was confirmed by the quantitative assay based on fluorescence dequenching described by Hoekstra et al. (1984), Biochemistry 23:5675-5681 and L. Lüscher et al. (1993), Arch. Virol. 130:317-326. The fluorescent probe octadecyl rhodamine B chloride (R18) (obtained from Molecular Probes Inc., Eugene, USA) was inserted at high densities into the membrane of DOSPER virosomes by adding the buffered OEG (C₁₂E₈) solution containing DOSPER and HA to a thin dry film of the fluorescent probe, followed by shaking for 5 to 10 minutes for dissolving the probe, then continuing as described above under "Preparation of DOSPER virosomes...". Dilution of the quenching rhodamine was observed by incubation of the rhodamine-labeled DOSPER virosomes with model liposomes (ratio of DOSPER/DOPC : liposomal phospholipid = 1 :20). The fluorescence was measured by a Perkin-Elmer 1000 spectrofluorimeter at 560 and 590 nm excitation and emission wavelengths, respectively.

### Example 2: Uptake of virosomes by cells

Incorporation of ³H-labeled plasmids into DOSPER virosomes:
Basically, incorporation of nucleic acid compounds into virosomes was accomplished by the following three methods:
1. Dialysis: The nucleic acid compounds were encapsulated during the formation of virosomes, wherein the detergent Octyl-POE (from Alexis Corp., Laeufelfingen, Switzerland) was removed by dialysis.
2. Biobeads: The nucleic acid compounds were encapsulated during formation of virosomes, wherein the detergent OEG was removed by microcarriers, e.g., Biobeads SM2.
3. Ultrasonication: The nucleic acid compounds were encapsulated by cationic lipids, preferably DOSPER, and the obtained nucleic acid-loaded liposomes or lipid complexes, preferably DOSPER liposomes or lipid complexes, were fused with DOSPER virosomes by ultrasonication.

Since best incorporation rates were obtained by the third method, most experiments were done using this fusion method for incorporation of nucleic acid material into the virosomes.

The plasmid pGEEN LANTERN™-I (GIBCO-BRL) was produced in E.coli in the presence of 3H-methylthymidine in order to obtain radioactively labeled plasmids for exact quantification of transfection efficiency. The specific activity of the plasmid was 6.625 x 10¹⁰ dpm/g (= 29.84 mCi/g).

11.6 µg of ³H labeled plasmid (17.5 µl) were dissolved in 480 µl HEPES-NaCL-buffer. After addition of 116 µg DOSPER (1µg/µl) the resulting mixture was treated by ultrasonicationfor 30 seconds and thereafter allowed to rest for 15 min. By this treatment lipid complexes made of DOSPER and plasmid form. Alternatively, instead of lipid complexes DOSPER liposomes may be produced by conventional methods, e.g. from a buffered solution of OEG which contains DOSPER and the plasmid, and subsequent removal of the detergent using dialysis or detergent- absorbing microcarriers, optionally followed by ultrasonication, resulting in DOSPER liposomes loaded with the plasmids.

To the solution containing the plasmid-loaded lipid complexes or liposomes 93 µl of pre-manufactured (e.g. according to Example 1) empty DOSPER virosomes are added. The mixture is ultrasonicated for 1 min to fuse the DOSPER virosomes with the liposomes or lipid complexes to incorporate the plasmids into the virosomes. This method of incorporating desired material into virosomes may also be applicable for incorporation of drugs or substances other than nucleic acid material, preferably for negatively charged substances.

Comparison of transfection efficiency of ³H-labeled plasmid by DOSPER virosomes and DOSPER iposomes:
33 µg of the labeled plasmid DNA were encapsulated into DOSPER virosomes and 11.7 µg into pre-manufactured DOSPER liposomes. Four different adherent cell lines, i.e., HeLa, 293T, COS-1 and NIH3T3 and additionally, the cell lines K562 and P3/NSI/1-Ag4-1 growing in suspension culture, were transfected with DOSPER virosomes and DOSPER liposomes.
5x10⁵ cells of each cell type were incubated in 500 µl (= 0.5 ml) of medium with 25 µl of either plasmid-containing DOSPER-virosomes or plasmid-containing DOSPER-liposomes solution for 2, 4, 6 and 24 h at 37ºC. The added aliquots of DOSPER-virosomes and DOSPER-liposomes contained 400 ng of labeled plasmid which corresponded to a radioactivity of 26,500 dpm (= 100%). After incubation, cells were washed, lysed and the uptaken radioactivity measured with a betacounter.

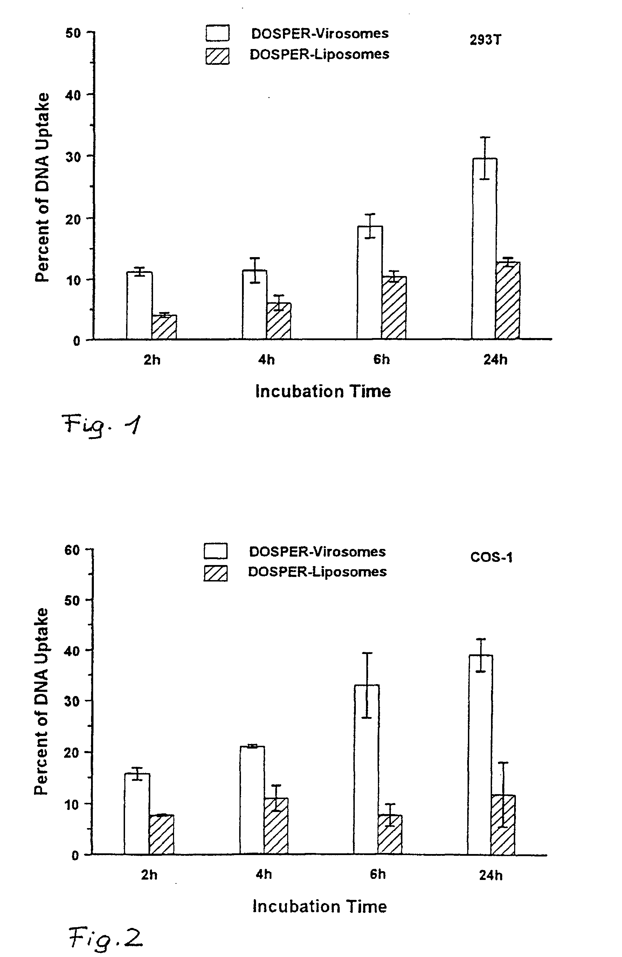

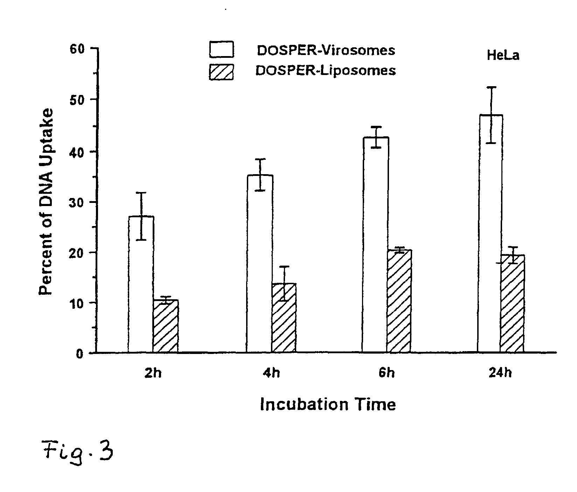

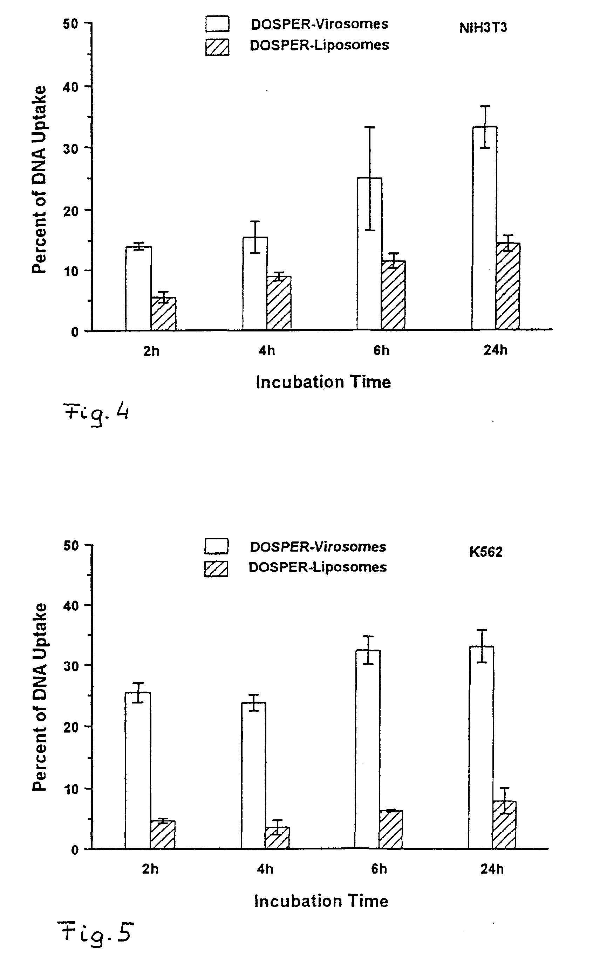

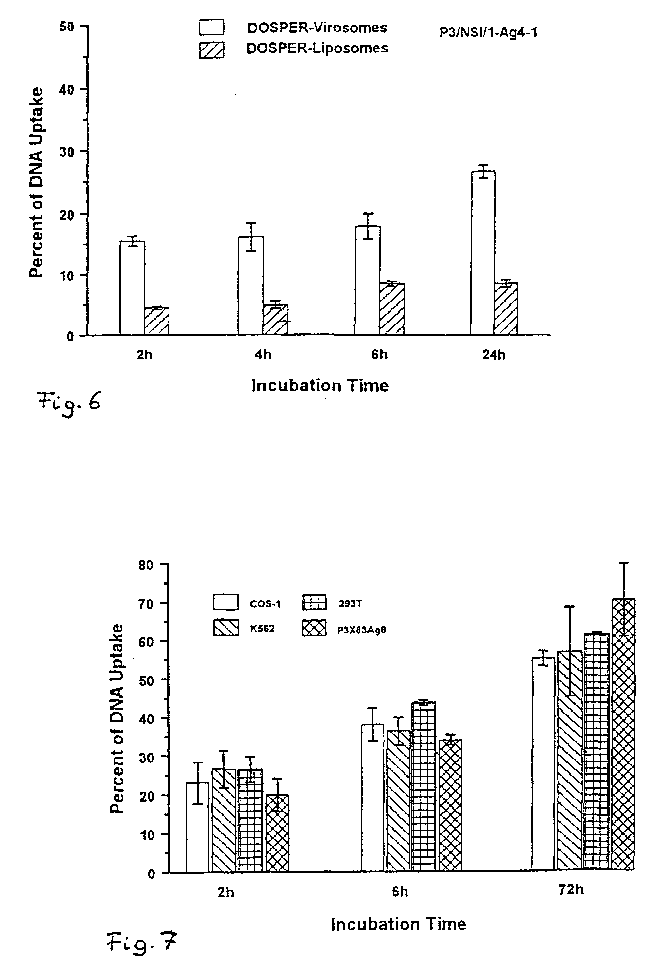

The influence of the length of time of transfection (incubation period) on the amount of uptaken plasmid-DNA was evaluated and the results are graphed in Figures 1 to 6. The Brief Description of the Figures above summarizes the cell lines in each of the Figures. Figures 1-6 show clearly that DOSPER-virosomes are superior in transfection to DOSPER-liposomes for all cell types and for all incubation times. Longer incubation times of 6 and 24 h with DOSPER-liposomes only resulted in very minimal increases of DNA uptake, whereas longer incubation times with DOSPER-virosomes led to considerably higher DNA uptake. This result suggests that the HA-mediated uptake of virosomes is far more efficient than the uptake induced by DOSPER-liposomes without the help of any viral binding ligands to cell receptors. It is interesting that in the case of DOSPER-virosomes, incubation times of 6-24 h did not lead to a saturation of DNA incorporation but to a further increase of DNA uptake, an effect which may be due to a rapid replacement of membrane receptors after their removing by receptor-mediated endocytosis of the virosomes on the cell surface. In general, it was found that adherent cell lines (growing in monolayers) were more efficiently transfected than the cell lines growing in suspension culture.

### Example 3: Time-dependent uptake of plasmids by eukaryotic cells.

Example 2 was repeated with another ³H-labeled plasmid, i.e. pcDNA3.1/His B/lacZ (Supplier: Invitrogen, Groningen, The Netherlands). Cell lines COS-1, K562, 293 T and P3X63Ag8 were transfected with DOSPER virosomes loaded with this plasmid and incubated for a period of 2, 4, and 72 hours. A comparison with DOSPER liposomes was not drawn in this experiment.

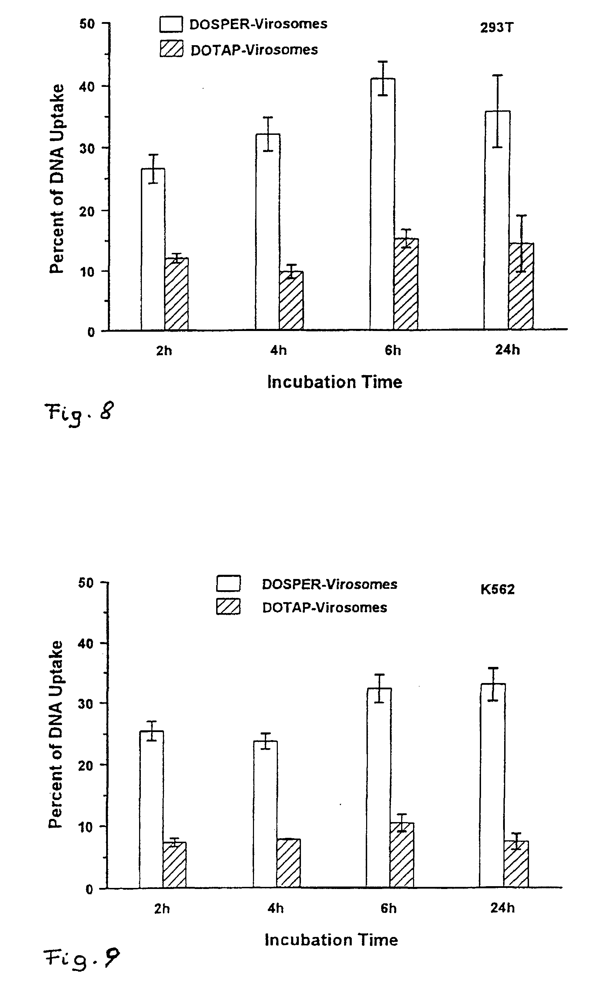

The results are graphed in Fig. 7. It can be taken therefrom that a prolongation of the time of incubation may well result in a further increase in cellular uptake of labeled plasmid when using the present virosomes. Indeed, using the present DOSPER virosomes as a high performance transfer system for nucleic acid material it is possible to deliver 60 - 80 % of the total amount of the nucleic acid (e.g. plasmid) input into the desired target cells. All tested cell lines were comparably susceptible to the virosome hence plasmid uptake.

### Example 4: A comparison of DOSPER virosomes to DOTAP virosomes

Virosomes penetrate mammalian cells by the HA-driven receptor-mediated mechanism kwown from influenza A virus. It was not expected that the chemical nature of the lipid bilayer of the virosome membrane could play an important role as long as the native status and the fusogenic activity of the HA was properly maintained. DOSPER virosomes and DOTAP virosomes were therefore believed to have approximately the same transfection efficiency for nucleic acid material into mammalian cells.
Surprisingly, however, experiments designed to compare the transfection efficiencies of DOSPER and DOTAP virosomes yielded a different result, i.e. remarkably deviating from the expectations. The experiments were done according to Examples 1 and 2 while using the tritium-labeled plasmid pcDNA3.1/His B/lacZ for encapsulation and transfection. The DOTAP virosomes were prepared according to Example 1 of WO97/41834 and incorporation of the plasmid was done as hereinbefore described using the same procedure as for the DOSPER virosomes. The same amount of ³H-labeled plasmid as described in Example 2 for DOSPER-virosomes was encapsulated into DOTAP-virosomes. Cell lines 293T, K562, Sp2 and HeLa were incubated with DOSPER and DOTAP virosomes under the same conditions as described above in Example 2.

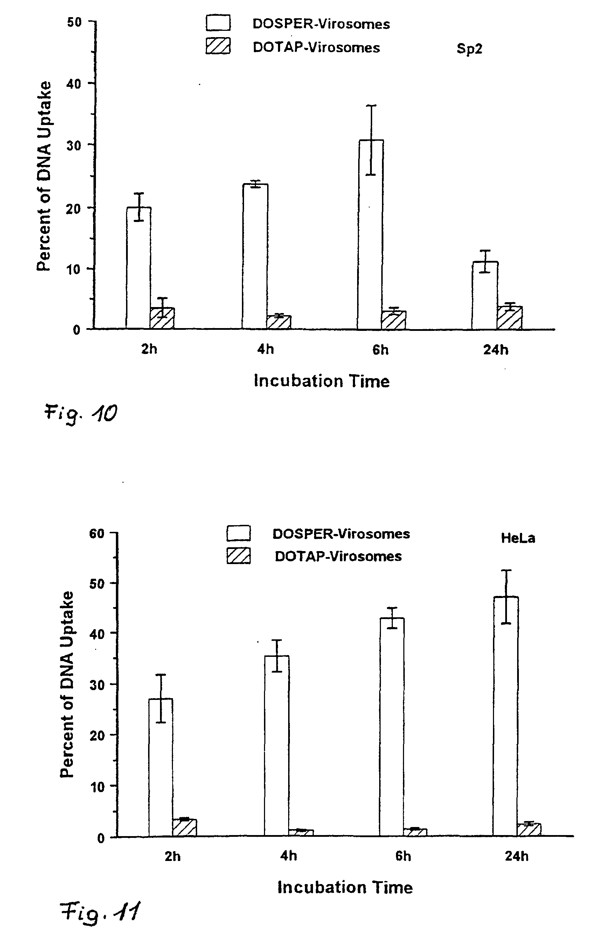

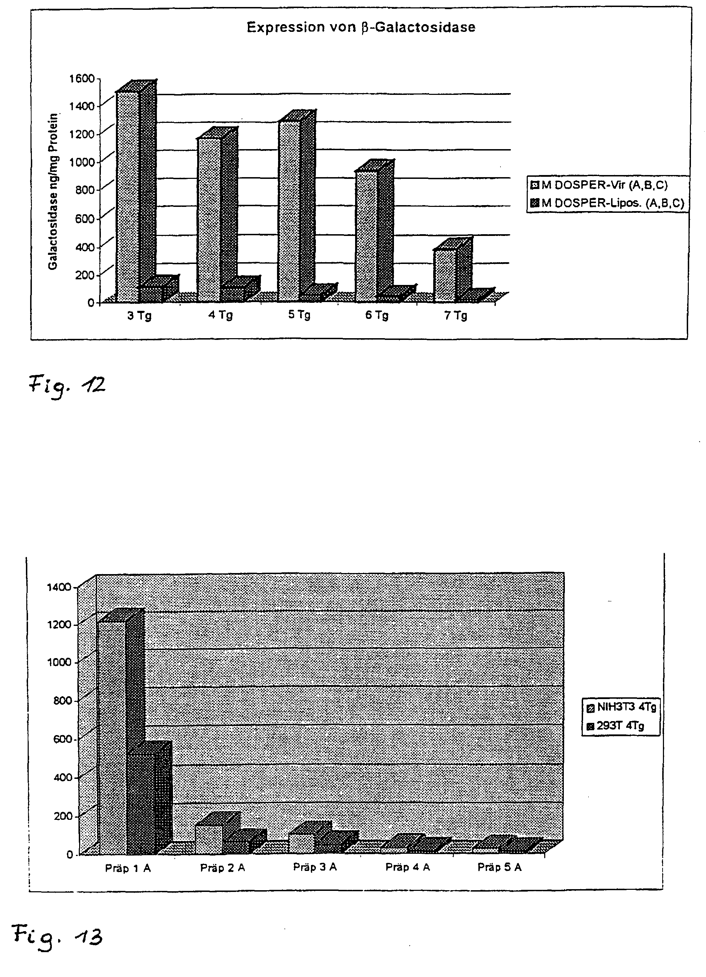

The results are graphed in Figures 8-11. They clearly show that the transfection efficiency of the DOSPER virosomes is at least twice as much as the one of the DOTAP virosomes and may even be up to ten times higher. Particularly with the cell lines growing in suspension culture such as the cell line Sp2 (Fig. 10) the difference in transfection efficency is most clearly visible. The reason therefor is not yet fully understood but it may at least partly be due to the different chemical nature of the two lipids, wherein the polar head of the DOSPER molecule contains four free amino groups while the polar head of the DOTAP molecule contains just one free amino group. We suppose, without being bound by theory, that encapsulation of negatively charged material, such as plasmids, into virosomes containing DOSPER may lead to higher amounts of useful virosomes. In contrast, encapsulation of plasmid into DOTAP-virosomes may result in a high number of defective vesicles with low transfection potential. A second hypothesis would implicate an enhancing effect of DOSPER during binding of the virosomes to the cell membrane of target cells, a viewpoint we cannot exclude.

The results thus demonstrate that use of the polycationic lipid DOSPER exhibits surprisingly superior transfection efficiency compared to the most frequently used cationic lipid DOTAP.

### Example 5: Gene expression after transfection with present virosomes

Quantitative determination using a β-Gal ELISA kit (supplier: Boehringer Mannheim) of the β-galactosidase expression performed by the target cells in the experiments of Example 3 that received the plasmid pcDNA3.1/His B/lacZ through virosomal transfection further confirmed that not only the transfection rate of the virosome-delivered plasmid was superior to the one of the liposome-delivered plasmid but also the expression thereof in the target cells. The experiments yielded a twentyfold to fourtyfold higher expression of β-galactosidase when using the DOSPER virosomes instead of the DOSPER liposomes for transfection of the plasmid into the target cells (Fig. 12).

It was also observerd, however, that there was a strong dependency of the level of β-galactosidase expression from the plasmid concentration within the virosomes:

**Table 2:**

| Composition of virosome preparations | | | | |
|---|---|---|---|---|
| | total amount of virosome-encapsulated plasmid [µg] | total amount of DOSPER in the virosomes [µg] | relative number of virosomes [arbitrary units] | ratio of plasmid / DOSPER [µg/µg] |
| Prep. 1 | 0.4 | 6.45 | 100 | 0.062 |
| Prep.2 | 0.2 | 4.45 | 100 | 0.045 |
| Prep.3 | 0.4 | 8.9 | 200 | 0.045 |
| Prep.4 | 0.13 | 3.78 | 100 | 0.034 |
| Prep.5 | 0.4 | 11.35 | 300 | 0.035 |

The virosomes of preparation 2 contain only half as much plasmid as the virosomes of preparation 1, while the number of virosomes is equal in both preparations.
Preparation 1 corresponds to the preferred virosome preparation that has been used for most of the experiments herein described.
Preparation 3 contains the same amount of plasmid, however distributed within a twofold number of virosomes.
Preparation 4 contains a still lower amount of plasmid encapsulated in the same number of virosomes as in preparations 1 and 2.
Preparation 5 contains the same amount of plasmid as preparations 1 and 3, however encapsulated within a number of virosomes that is three times as high as in preparation 1.

The results graphed in Figure 13 testify that the empirically derived, preferred DOSPER virosome formulation was rather optimal, and further that lowering the plasmid concentration in preparations 2, 3 and 4 did not result in an augmented level of expression. The hypothesis that an overload of the target cells through transfection with virosome preparations similar to the above Preparation 1 could possibly have inhibited the expression was therefore no longer tenable.

### Abbreviations used in the description

- 2'-OMe: 2'-O methyl
- CALLA: common acute lymphoblastic leukemia antigen
- CAT: chloramphenicol acteyltransferase
- dpm: disintegrations per minute
- DOSPER: 1,3-dioleoyloxy-2-(6-carboxy-spermyl)-propylamide
- DOTAP: N-[(1,2,3-dioleoyloxy)-propyl]-N,N,N-trimethylammoniummethyl-sulfate
- FITC-OPT: fluorescein isothiocyanate-labeled oligodeoxyribonucleotide phosphorothioate
- G418: Geneticin® disulfat (antibiotic G418)
- HA: hemagglutinin
- MPB.PE: N-[4-(p-maleimido)-phenylbutyryl]-phosphatidylethanolamine ( = a crosslinker-phospholipid complex)
- msc: mixed sequence control
- NA: neuraminidase
- Octyl-POE: n-octyl-oligo-oxyethylene
- ODN: oligodeoxynucleotides
- OEG: octaethyleneglycol monododecylether (C12E8)
- OPT: oligodeoxyribonucleotide phosphorothioate(s)
- PC: phosphatidylcholine
- PE: phosphatidylethanolamine
- PNA: peptide nucleic acid
- SCLC: small cell lung cancer
- SV40: Simian virus 40

## Claims

1. A lipid vesicle having a bilayer membrane that has a positive net charge at physiological pH and wherein the vesicle further comprises at least one active fusogenic peptide that is a non-Sendai viral hemagglutinin that causes the vesicles to be internalized by target cells through receptor-mediated phagocytosis or endocytosis,
**characterized in that** the vesicle membrane comprises 5 to 30 % by weight, based on total membrane lipids, of 1,3-dioleoyloxy-2-(6-carboxy-spermyl)-propylamide (DOSPER), and a balance of 95 to 70 % by weight of other lipids comprising phosphatidylcholine (PC) or a derivative thereof and optionally phosphatidylethanolamine (PE) and/or cationic lipids other than DOSPER.

2. The vesicle according to claim 1, **characterized in that** it further contains a desired drug or substance for delivery to target cells, said desired drug or substance preferably being negatively charged and particularly being selected from the group consisting of a dye, a tracer substance, a cosmetic agent, a pharmaceutically or biologically active substance, and a nucleic acid material.

3. The vesicle according to claim 2, **characterized in that** the desired drug or substance is a nucleic acid material selected from the group consisting of short chain DNA or RNA, deoxyribonucleotides, oligodeoxyribonucleotides, oligodeoxyribonucleotide selenoates, oligodeoxyribonucleotide phosphorothioates, oligodeoxyribonucleotide phosphoramidates, oligodeoxyribonucleotide methylphosphonates, peptide nucleic acids, ribonucleotides, oligoribonucleotides, oligoribonucleotide phosphorothioates, 2'-OMe-oligoribonucleotide phosphates, 2'-OMe-oligoribonucleotide phosphorothioates, ribozymes, genes, plasmids and vectors.

4. The vesicle according to any one of claims 1 to 3, **characterized in that** the desired drug or substance is a nucleic acid material and the vesicle comprises said nucleic acid material in an amount ranging from 0.03 to 0.1, preferably 0.045 to 0.065, µg nucleic acid material per 1 µg DOSPER.

5. The vesicle according to any one of claims 1 to 4, **characterized in that** the average vesicle diameter is in a range of 120 - 180 nm.

6. The vesicle according to any one of claims 1 to 5, **characterized in that** it further comprises at least one cell-specific marker attached to the vesicle membrane.

7. The vesicle according to claim 6, **characterized in that** the vesicle membrane further comprises phosphatidylethanolamine (PE) and a bifunctional crosslinker which is linked with one of its binding sites to a free amino group of the PE and with its other binding site to the cell-specific marker.

8. The vesicle according to claim 6 or 7, **characterized in that** the cell-specific marker is a biologically active protein for binding to a receptor of target cells selected from the group consisting of an antibody, an antibody fragment, a cytokine and a growth factor,

9. The vesicle according to any one of claims 1 to 8, **characterized in that** the hemagglutinin is derived from a virus selected from the group consisting of rhabdovirus, parainfluenza virus type III, Semliki Forest virus and togavirus.

10. The vesicle according to any one of claims 1 to 9, **characterized in that** the vesicle membrane comprises DOSPER in a concentration of 10 to 30 %, preferably 20 to 25 % by weight, based on total membrane lipids.

11. The vesicle according to any one of claims 1 to 10, **characterized in that** the vesicle membrane comprises, based on total membrane lipids, 25 % by weight of DOSPER and 75 % by weight of PC, preferably dioleoylphosphatidylcholine (DOPC).

12. A composition comprising a plurality of vesicles defined in any one of claims 1 to 11 in combination with a suitable carrier, for use in cosmetic, pharmaceutical or diagnostic applications.

13. A process for the manufacture of a lipid vesicle as defined in claim 1, **characterized in that** it comprises the steps of
(a) preparing a buffer solution that comprises a non-ionic detergent and that further comprises DOSPER and other lipids and at least one active fusogenic peptide that is a non-Sendai viral hemagglutinin that causes the vesicles to be internalized by target cells through receptor-mediated phagocytosis or endocytosis;
b) adjusting the lipid concentrations to - based on total membrane lipids - 5 to 30 % by weight of DOSPER and to a balance of 95 to 70 % by weight of said other lipids comprising phosphatidylcholine (PC) or a derivative thereof and optionally phosphatidylethanolamine (PE) and/or cationic lipids other than DOSPER; and
(c) removing the detergent by dialysis or by treating the solution with microcarrier beads, resulting in the formation of said positively charged lipid bilayer vesicles.

14. The process according to claim 13, **characterized in that** it further comprises a step of incorporating into the vesicles a quantity of a desired drug or substance for delivery to target cells, said desired drug or substance preferably being negatively charged and particularly being selected from the group consisting of a dye, a tracer substance, a cosmetic agent, a pharmaceutically or biologically active substance, and a nucleic acid material.

15. The process according to claim 13 or 14, **characterized in that** it further comprises a step of preparing conjugate molecule complexes consisting of phosphatidylethanolamine (PE), a bifunctional crosslinker, and a cell-specific marker in a way such that the bifunctional crosslinker with one binding site links to the amino group of PE and with another one to a thiol group of the cell-specific marker, removing unconjugated material and adding the molecule complexes to the solution in step a).

16. The process according to claim 14 or 15, **characterized in that** incorporation of the desired drug or substance is accomplished by adding to the solution in step (a) the desired drug or substance, whereafter in step (c) vesicles containing said drug or substance are formed.

17. The process according to claim 14 or 15, **characterized in that** incorporation of the desired drug or substance into the vesicles is accomplished by adding to the vesicles resulting from step (c) the desired drug or substance, sonicating the mixture to integrate the drug or substance into the vesicles, and removing non-integrated material, preferably by gel filtration.

18. The process according to claim 14 or 15, **characterized in that** incorporation of the desired drug or substance into the vesicles is accomplished by adding to the vesicles resulting from step (c) positively charged lipid complexes or liposomes loaded with the desired drug or substance and sonicating the resulting mixture to fuse the lipid complexes or liposomes with said vesicles to integrate the drug or substance into said vesicles.

19. The process according to claim 18, **characterized in that** the positively charged lipid complexes or liposomes comprise 50 to 100% by weight, based on their total lipid contents, of DOSPER lipid.

20. The process according to any one of claims 15 to 19, wherein the cell-specific marker is selected from the group consisting of an antibody, an antibody fragment, a cytokine and a growth factor.

21. The process according to any one of claims 14 to 20, **characterized in that** the desired drug or substance is a nucleic acid material selected from the group consisting of short chain DNA or RNA, deoxyribonucleotides, oligodeoxyribonucleotides, oligodeoxyribonucleotide selenoates, oligodeoxyribonucleotide phosphorothioates, oligodeoxyribonucleotide phosphoramidates, oligodeoxyribonucleotide methylphosphonates, peptide nucleic acids, ribonucleotides, oligoribonucleotides, oligoribonucleotide phosphorothioates, 2'-OMe-oligoribonucleotide phosphates, 2'-OMe-oligoribonucleotide phosphorothioates, ribozymes, genes, plasmids and vectors.

22. The process according to claim 21, **characterized in that** the nucleic acid material is incorporated into the virosomes at a concentration of 0.03 to 0.1, preferably 0.045 to 0.065, µg nucleic acid material per 1 µg DOSPER lipid.

23. The process according to any one of claims 13 to 22, **characterized in that** the non-ionic detergent is octaethyleneglycol monododecylether (C₁₂E₈)() or n-octyl-oligooxyethylene.

24. The process according to any one of claims 15 to 23, **characterized in that** the crosslinking agent is a heterobifunctional organic molecule having at least one maleimido and at least one carboxyl group, and preferably is selected from the group consisting of bis-N-succinimidyl derivatives and photactivatable succinimidyl derivatives.

25. Use of a vesicle defined in claim 1 for the manufacture of a composition for cosmetic, diagnostic or medical applications, particularly for delivering a desired drug or substanceto resting or proliferating target cells.

26. Use according to claim 25, **characterized in that** said target cells are incubated in vitro in the presence of said vesicles and that said incubated target cells are optionally tansferable to an organism in vivo.

27. Use according to claim 25 or 26, **characterized in that** the target cells are selected from the group consisting of cancer cells, leukemic cells and virally infected cells.

28. Use according to claim 27, **characterized in that** metabolic or proliferative activity of said target cells is reduced upon incubation in the presence of said vesicles.

29. Use according to any one of claims 25 to 28, **characterized in that** said desired drug or substance is a nucleic acid material which comprises at least one antisense oligonucleotide, preferably an antisense oligonucleotide that is targeting protooncogene or oncogene encoded mRNA.

## Patentansprüche

1. Lipidvesikel mit einer Doppelschichtmembran, die bei physiologischen pH-Werten eine positive Nettoladung besitzt, und worin das Vesikel weiter zumindest ein aktives fusogenes Peptid umfasst, das ein Nicht-Sendaivirus-Hämagglutinin ist, das die Vesikel durch rezeptor-vermittelte Phagocytose oder Endocytose veranlasst, durch Targetzellen internalisiert zu werden, **dadurch gekennzeichnet, dass** die Vesikelmembran relativ zu den gesamten Membranlipiden 5 bis 30 Gewichtsprozent an 1,3-Dioleoyloxy-2-(6-carboxyspermyl)propylamid (DOSPER) und eine Differenz von 95 bis 70 Gewichtsprozent an anderen Lipiden enthält, darunter Phosphatidylcholin (PC) oder einen Abkömmling davon sowie wahlweise Phosphatidylethanolamin (PE) und/oder kationische Lipide ausser DOSPER.

2. Vesikel nach Anspruch 1, **dadurch gekennzeichnet, dass** es weiter eine erwünschte Arznei oder Substanz zur Abgabe an Targetzellen enthält, wobei diese Arznei bzw. Substanz bevorzugt negativ geladen und speziell aus der Gruppe ausgewählt ist, die aus einem Farbstoff, einer Tracersubstanz, einem Kosmetikum, einer pharmazeutisch oder biologisch aktiven Substanz und einem Nucleinsäurematerial besteht.

3. Vesikel nach Anspruch 2, **dadurch gekennzeichnet, dass** die gewünschte Arznei bzw. Substanz ein Nucleinsäurematerial ist, das aus der Gruppe ausgewählt ist, die aus kurzkettigen DNA oder RNA, Desoxyribonucleotiden, Oligodesoxyribonucleotiden, Oligodesoxyribonucleotid-selenoaten, Oligodesoxyribonucleotid-phosphorothioaten, Oligodesoxyribonucleotid-phosphoramidaten, Oligodesoxyribonucleotid-methylphosphonaten, Peptidnucleinsäuren, Ribonucleotiden, Oligoribonucleotiden, Oligoribonucleotid-phosphorothioaten, 2'-OMe-Oligoribonucleotid-phosphaten, 2'-OMe-Oligoribonucleotid-phosphorothioaten, Ribozymen, Genen, Plasmiden und Vektoren besteht.

4. Vesikel nach einem beliebigen der Ansprüche 1 bis 3, **dadurch gekennzeichnet, dass** die erwünschte Arznei bzw. Substanz ein Nucleinsäurematerial ist und das Vesikel dieses Nucleinsäurematerial in einer Menge zwischen 0,03 und 0,1, bevorzugt zwischen 0,045 und 0,065 µg Nucleinsäurematerial pro 1 µg DOSPER enthält.

5. Vesikel nach einem beliebigen der Ansprüche 1 bis 4, **dadurch gekennzeichnet, dass** der durchschnittliche Vesikeldurchmesser im Bereich von 120 bis 180 nm liegt.

6. Vesikel nach einem beliebigen der Ansprüche 1 bis 5, **dadurch gekennzeichnet, dass** es weiter zumindest einen an die Vesikelmembran angehängten, zellspezifischen Marker umfasst.

7. Vesikel nach Anspruch 6, **dadurch gekennzeichnet, dass** die Vesikelmembran weiter Phosphatidylethanolamin (PE) und einen bifunktionellen Crosslinker umfasst, der mit einer seiner Bindungsstellen an eine freie Aminogruppe des PE und mit seiner anderen Bindungsstelle an den zellspezifischen Marker gebunden ist.

8. Vesikel nach Anspruch 6 oder 7, **dadurch gekennzeichnet, dass** der zellspezifische Marker ein biologisch aktives Protein zur Bindung an einen Rezeptor von Targetzellen ist, das aus der Gruppe ausgewählt ist, die aus einem Antikörper, einem Antikörperfragment, einem Cytokin und einem Wachstumsfaktor besteht.

9. Vesikel nach einem beliebigen der Ansprüche 1 bis 8, **dadurch gekennzeichnet, dass** das Hämagglutinin von einem Virus abgeleitet ist, der aus der Gruppe ausgewählt ist, die aus Rhabdovirus, Parainfluenzavirus des Typs 3, Semliki Forest-Virus und Togavirus besteht.

10. Vesikel nach einem beliebigen der Ansprüche 1 bis 9, **dadurch gekennzeichnet, dass** die Vesikelmembran relativ zu den gesamten Membranlipiden DOSPER in einer Konzentration von 10 bis 30 Gewichtsprozent, bevorzugt von 20 bis 25 Gewichtsprozent enthält.

11. Vesikel nach einem beliebigen der Ansprüche 1 bis 10, **dadurch gekennzeichnet, dass** die Vesikelmembran relativ zu den gesamten Membranlipiden 25 Gewichtsprozent DOSPER und 75 Gewichtsprozent PC, bevorzugt Dioleoylphosphatidylcholin (DOPC), enthält.

12. Zusammensetzung aus einer Mehrzahl von Vesikeln, wie in einem beliebigen der Ansprüche 1 bis 11 definiert, in Kombination mit einem geeigneten Träger zur Verwendung in kosmetischen, pharmazeutischen oder diagnostischen Anwendungen.

13. Verfahren zur Herstellung eines Lipidvesikels, wie in Anspruch 1 definiert, **dadurch gekennzeichnet, dass** es die Schritte umfasst,
a) eine Pufferlösung zuzubereiten, die ein nichtionisches Tensid enthält und die weiter DOSPER und andere Lipide sowie zumindest ein aktives, fusogenes Peptid enthält, das ein Nicht-Sendaivirus-Hämagglutinin ist und die Vesikel durch rezeptor-vermittelte Phagocytose oder Endocytose veranlasst, durch Targetzellen intemalisiert zu werden;
b) die Lipidkonzentrationen relativ zu den gesamten Membranlipiden auf 5 bis 30 Gewichtsprozent DOSPER und eine Differenz von 95 bis 70 Gewichtsprozent dieser anderen Lipide, darunter Phosphatidylcholin (PC) oder einen Abkömmling davon sowie wahlweise Phosphatidylethanolamin (PE) und/oder kationische Lipide ausser DOSPER, einzustellen; und
c) das Tensid durch Dialyse oder durch Behandlung der Lösung mit Microcarrier-beads zu entfernen, was zur Bildung dieser positiv geladenen Lipid-Doppelschichtvesikel führt.

14. Verfahren nach Anspruch 13, **dadurch gekennzeichnet, dass** es weiter einen Schritt umfasst, in die Vesikel eine Menge einer erwünschten Arznei oder Substanz zur Abgabe an Targetzellen einzubauen, wobei diese Arznei bzw. Substanz bevorzugt negativ geladen und speziell aus der Gruppe ausgewählt ist, die aus einem Farbstoff, einer Tracersubstanz, einem Kosmetikum, einer pharmazeutisch oder biologisch aktiven Substanz und einem Nucleinsäurematerial besteht.

15. Verfahren nach Anspruch 13 oder 14, **dadurch gekennzeichnet, dass** es weiter einen Schritt umfasst, konjugierte Molekülkomplexe, die aus Phosphatidylethanolamin (PE), einem bifunktionellen Crosslinker und einem zellspezifischen Marker bestehen, derart herzustellen, dass der bifunktionelle Crosslinker mit einer Bindungsstelle an die Aminogruppe von PE und mit einer anderen Bindungsstelle an eine Thiolgruppe des zellspezifischen Markers bindet, nicht konjugiertes Material abzutrennen und die Molekülkomplexe zur Lösung im Schritt (a) hinzuzufügen.

16. Verfahren nach Anspruch 14 oder 15, **dadurch gekennzeichnet, dass** der Einbau der erwünschten Arznei bzw. Substanz durch Zugabe der erwünschten Arznei bzw. Substanz zur Lösung in Schritt (a) erreicht wird, wonach im Schritt (c) Vesikel gebildet werden, die die erwünschte Arznei bzw. Substanz enthalten.

17. Verfahren nach Anspruch 14 oder 15, **dadurch gekennzeichnet, dass** der Einbau der erwünschten Arznei bzw. Substanz in die Vesikel erreicht wird, indem zu den aus Schritt (c) sich ergebenden Vesikeln die erwünschte Arznei bzw. Substanz hinzugefügt wird, das Gemisch mit Ultraschall behandelt wird, um die Arznei bzw. Substanz in die Vesikel zu integrieren, und nichtintegriertes Material bevorzugt mittels Gelfiltration abgetrennt wird.

18. Verfahren nach Anspruch 14 oder 15, **dadurch gekennzeichnet, dass** der Einbau der erwünschten Arznei bzw. Substanz in die Vesikel erreicht wird, indem zu den aus Schritt (c) sich ergebenden Vesikeln positiv geladene Lipidkomplexe oder Liposome hinzugefügt werden, die mit der erwünschten Arznei bzw. Substanz beladen sind, und das anfallende Gemisch mit Ultraschall behandelt wird, um die Lipidkomplexe oder Liposomen mit diesen Vesikeln zu verschmelzen und die Arznei bzw. Substanz in diese Vesikel zu integrieren.

19. Verfahren nach Anspruch 18, **dadurch gekennzeichnet, dass** die positiv geladenen Lipidkomplexe oder Liposomen relativ zum Gesamtgehalt an Lipiden 50 bis 100 Gewichtsprozent des DOSPER-Lipids enthalten.

20. Verfahren nach einem beliebigen der Ansprüche 15 bis 19, worin der zellspezifische Marker aus der Gruppe ausgewählt ist, die aus einem Antikörper, einem Antikörperfragment, einem Cytokin und einem Wachstumsfaktor besteht.

21. Verfahren nach einem beliebigen der Ansprüche 14 bis 20, **dadurch gekennzeichnet, dass** die erwünschte Arznei bzw. Substanz ein Nucleinsäurematerial ist, das aus der Gruppe ausgewählt ist, die aus kurzkettigen DNA oder RNA, Desoxyribonucleotiden, Oligodesoxyribonucleotiden, Oligodesoxyribonucleotid-selenoaten, Oligodesoxyribonucleotid-phosphorothioaten, Oligodesoxyribonucleotid-phosphoramidaten, Oligodesoxyribonucleotid-methylphosphonaten, Peptidnucleinsäuren, Ribonucleotiden, Oligoribonucleotiden, Oligoribonucleotid-phosphorothioaten, 2'-OMe-Oligoribonucleotid-phosphaten, 2'-OMe-Oligoribonucleotid-phosphorothioaten, Ribozymen, Genen, Plasmiden und Vektoren besteht.

22. Verfahren nach Anspruch 21, **dadurch gekennzeichnet, dass** das Nucleinsäurematerial in einer Konzentration zwischen 0,03 und 0,1, bevorzugt zwischen 0,045 und 0,065 µg Nucleinsäurematerial pro 1 µg DOSPER-Lipid in die Virosomen eingebaut wird.

23. Verfahren nach einem beliebigen der Ansprüche 13 bis 22, **dadurch gekennzeichnet, dass** das nichtionische Tensid Octaethylenglykol-monododecylether (C₁₂E₈)() oder *n*-Octyl-oligooxyethylen ist.

24. Verfahren nach einem beliebigen der Ansprüche 15 bis 23, **dadurch gekennzeichnet, dass** das Vernetzungsmittel ein hetero-bifunktionelles organisches Molekül ist, das zumindest eine Maleimido- und zumindest eine Carboxylgruppe besitzt und bevorzugt aus der Gruppe ausgewählt ist, die aus Bis-*N*-succinimidylabkömmlingen und photoaktivierbaren Succinimidylabkömmlingen besteht.

25. Verwendung eines in Anspruch 1 definierten Vesikels für die Herstellung einer Zusammensetzung für kosmetische, diagnostische oder medizinische Anwendungen, insbesondere zur Abgabe einer erwünschten Arznei oder Substanz an im Ruhezustand befindliche oder proliferierende Targetzellen.

26. Verwendung nach Anspruch 25, **dadurch gekennzeichnet, dass** diese Targetzellen *in vitro* in Gegenwart dieser Vesikel inkubiert werden und dass diese inkubierten Targetzellen wahlweise zu einem Organismus *in vivo* überführt werden können.

27. Verwendung nach Anspruch 25 oder 26, **dadurch gekennzeichnet, dass** die Targetzellen aus der Gruppe ausgewählt sind, die aus Krebszellen, leukämischen Zellen und virusinfizierten Zellen besteht.

28. Verwendung nach Anspruch 27, **dadurch gekennzeichnet, dass** die Stoffwechseloder proliferative Aktivität dieser Targetzellen nach Inkubation in Gegenwart dieser Vesikel vermindert ist.

29. Verwendung nach einem beliebigen der Ansprüche 25 bis 28, **dadurch gekennzeichnet, dass** die erwünschte Arznei bzw. Substanz ein Nukleinsäurematerial ist, das zumindest ein Antisense-Oligonucleotid umfasst, bevorzugt ein Antisense-Oligonucleotid, das auf proto-onkogen- oder onkogen-kodierte mRNA abzielt.

## Revendications

1. Vésicule lipidique comprenant une membrane à double couche qui présente une charge nette positive à un pH physiologique et dans laquelle la vésicule comprend en outre au moins un peptide fusogène actif qui est une hémagglutinine virale qui n'est pas de type Sendai, qui amène les vésicules à être internalisées par des cellules cibles par l'intermédiaire d'une phagocytose ou d'une endocytose médiée par un récepteur, **caractérisée en ce que** la membrane de la vésicule comprend 5 à 30 % en poids, sur la base de tous les lipides de la membrane, de 1,3-dioléoyloxy-2-(6-carboxyspermyl)-propylamide (DOSPER) et un complément de 95 à 70 % en poids d'autres lipides comprenant la phosphatidylcholine (PC) ou un dérivé de celle-ci et facultativement la phosphatidyléthanolamine (PE) et/ou des lipides cationiques autres que l'amide DOSPER.

2. Vésicule selon la revendication 1, **caractérisée en ce qu'**elle contient en outre un médicament ou une substance souhaité destiné à une délivrance à des cellules cibles, ledit médicament ou ladite substance souhaité étant de préférence chargé négativement et étant choisi de préférence parmi le groupe constitué d'une teinture, d'une substance de traceur, d'un agent cosmétique, d'une substance pharmaceutiquement ou biologiquement active et d'un matériau d'acide nucléique.

3. Vésicule selon la revendication 2, **caractérisée en ce que** le médicament ou la substance souhaité est d'un matériau d'acide nucléique choisi parmi le groupe constitué d'un ADN ou d'un ARN à chaîne courte de désoxyribonucléotides, d'oligodésoxyribonucléotides, de sélénoates d'oligodésoxyribonucléotides, de phosphorothioates d'oligodésoxyribonucléotides, de phosphoramidates d'oligodésoxyribonucléotides, de méthylphosphonates d'oligodésoxyribonucléotides, d'-acides nucléiques peptidiques, de ribonucléotides, d'oligoribonucléotides, de phosphorothioates d'oligoribonucléotides, de phosphates de 2'-Ome-oligoribonucléotides, de phosphorothioates de 2'-Ome-oligoribonucléotides, de ribozymes, de gènes, de plasmides et de vecteurs.

4. Vésicule selon l'une quelconque des revendications 1 à 3, **caractérisée en ce que** le médicament ou la substance souhaité est un matériau d'acide nucléique et la vésicule comprend ledit matériau d'acide nucléique dans une proportion allant de 0,03 à 0,1, de préférence de 0,045 à 0,065 µg de matériau d'acide nucléique pour 1 µg d'amide DOSPER.

5. Vésicule selon l'une quelconque des revendications 1 à 4, **caractérisée en ce que** le diamètre moyen de la vésicule se situe dans une plage de 120 à 180 nm.

6. Vésicule selon l'une quelconque des revendications 1 à 5, **caractérisée en ce qu'**elle comprend en outre au moins un marqueur spécifique de cellules fixé à la membrane de la vésicule.

7. Vésicule selon la revendication 6, **caractérisée en ce que** la membrane de la vésicule comprend en outre de la phosphatidyléthanolamine (PE) et un agent de liaison croisée bifonctionnel qui est lié à l'un de ses sites de liaison à un groupement amino libre de l'amine PE et avec son autre site de liaison au marqueur spécifique de cellules.

8. Vésicule selon la revendication 6 ou 7, **caractérisée en ce que** le marqueur spécifique de cellules est une protéine biologiquement active destinée à une liaison à un récepteur de cellules cibles choisie parmi le groupe constitué d'un anticorps, d'un fragment d'anticorps, d'une cytokine et d'un facteur de croissance.

9. Vésicule selon l'une quelconque des revendications 1 à 8, **caractérisée en ce que** l'hémagglutinine est dérivée d'un virus choisi parmi le groupe constitué du rhabdovirus, du virus parainfluenza type III, du virus Semliki Forest et du togavirus.

10. Vésicule selon l'une quelconque des revendications 1 à 9, **caractérisée en ce que** la membrane de la vésicule comprend l'amide DOSPER à une concentration de 10 à 30 %, de préférence 20 à 25 % en poids, sur la base de tous les lipides de la membrane.

11. Vésicule selon l'une quelconque des revendications 1 à 10, **caractérisée en ce que** la membrane de la vésicule comprend, sur la base de tous les lipides de la membrane, 25 % en poids d'amide DOSPER et 75 % en poids de phosphatidylcholine (PC), de préférence la dioléoylphosphatidylcholine (DOPC).

12. Composition comprenant une pluralité de vésicules définies dans l'une quelconque des revendications 1 à 11, en combinaison avec un support approprié, en vue d'une utilisation dans des applications cosmétiques, pharmaceutiques ou de diagnostic.

13. Procédé de fabrication d'une vésicule lipidique selon la revendication 1, **caractérisé en ce qu'**il comprend les étapes consistant à
(a) préparer une solution tampon qui comprend un détergent non ionique et qui comprend en outre l'amide DOSPER et d'autres lipides et au moins un peptide fusogène actif sous la forme d'une hémagglutinine virale qui n'est pas de type Sendai, et qui amène les vésicules à être internalisées par des cellules cibles par l'intermédiaire d'une phagocytose ou d'une endocytose médiée par un récepteur,
(b) ajuster les concentrations en lipides à, sur la base des lipides totaux de la membrane, 5 à 30 % en poids d'amide DOSPER et à un complément de 95 à 70 % en poids desdits autres lipides comprenant la phosphatidylcholine (PC) ou un dérivé de celle-ci et facultativement la phosphatidyléthanolamine (PE) et/ou des lipides cationiques autres que l'amide DOSPER, et
(c) éliminer le détergent par dialyse ou en traitant la solution avec des perles de microsupport, ce qui résulte en la formation desdites vésicules lipidiques à double couche chargées positivement.

14. Procédé selon la revendication 13, **caractérisé en ce qu'**il comprend en outre une étape consistant à incorporer dans les vésicules une certaine quantité d'un médicament ou d'une substance souhaité en vue d'une délivrance à des cellules cibles, ledit médicament ou ladite substance souhaité étant de préférence négativement chargé et étant en particulier choisi parmi le groupe constitué d'une teinture, d'une substance de traceur, d'un agent cosmétique, d'une substance pharmaceutiquement ou biologiquement active et d'un matériau d'acide nucléique.

15. Procédé selon la revendication 13 ou 14, **caractérisé en ce qu'**il comprend en outre une étape consistant à préparer des complexes de molécules conjuguées constitués de phosphatidyléthanolamine (PE), d'un agent de liaison croisée bifonctionnel et d'un marqueur spécifique de cellules de telle manière que l'agent de liaison croisée bifonctionnel, avec un premier site de liaison, se lie au groupement amino de l'amine PE et, avec un autre, à un groupement thiol du marqueur spécifique de cellules, éliminant le matériau non conjugué et ajoutant les complexes de molécules à la solution de l'étape (a).

16. Procédé selon la revendication 14 ou 15, **caractérisé en ce que** l'incorporation du médicament ou de la substance souhaité est réalisée en ajoutant à la solution de l'étape (a) le médicament ou la substance souhaité, à la suite de quoi à l'étape (c), les vésicules contenant ledit médicament ou ladite substance sont formées.

17. Procédé selon la revendication 14 ou 15, **caractérisé en ce que** l'incorporation du médicament ou de la substance souhaité dans les vésicules est réalisée en ajoutant aux vésicules résultant de l'étape (c) le médicament ou la substance souhaité, en soumettant le mélange à une sonication pour intégrer le médicament ou la substance dans les vésicules et en éliminant le matériau non intégré, de préférence par filtration sur gel.

18. Procédé selon la revendication 14 ou 15, **caractérisé en ce que** l'incorporation du médicament de la substance souhaité dans les vésicules est réalisée en ajoutant aux vésicules résultant de l'étape (c) les complexes lipidiques chargés positivement ou les liposomes chargés par le médicament ou la substance souhaité et en soumettant le mélange résultant à une sonication pour fusionner les complexes lipidiques ou les liposomes avec lesdites vésicules pour intégrer le médicament ou la substance dans lesdites vésicules.

19. Procédé selon la revendication 18, **caractérisé en ce que** les complexes lipidiques chargés positivement ou les liposomes comprennent 50 à 100 % en poids, sur la base de leurs teneurs totales en lipides, du lipide DOSPER.

20. Procédé selon l'une quelconque des revendications 15 à 19, dans lequel le marqueur spécifique de cellules est choisi parmi le groupe constitué d'un anticorps, d'un fragment d'anticorps, d'une cytokine et d'un facteur de croissance.

21. Procédé selon l'une quelconque des revendications 14 à 20, **caractérisé en ce que** le médicament ou la substance souhaité est un matériau d'acide nucléique choisi parmi le groupe constitué d'un ADN ou d'un ARN à chaîne courte, de désoxyribonucléotides, d'oligodésoxyribonucléotides, de sélénoates d'oligodésoxyribonucléotides, de phosphorothioates d'oligodésoxyribonucléotides, de phosphoramidates d'oligodésoxy-ribonucléotides, de méthylphosphonates d'oligodésoxyribonucléotides, d'acides nucléiques peptidiques, de ribonucléotides, d'oligoribonucléotides, de phosphorothioates d'oligoribonucléotides, de phosphates de 2'-OMe-oligoribonucléotides, de phosphorothioates de 2'-OMe-oligoribonucléotides, de ribozymes, de gènes, de plasmides et de vecteurs.

22. Procédé selon la revendication 21, **caractérisé en ce que** le matériau d'acide nucléique est incorporé dans les virosomes à une concentration de 0,03 à 0,1, de préférence de 0,045 à 0,065 µg de matériau d'acide nucléique pour 1 µg de lipide DOSPER.

23. Procédé selon l'une quelconque des revendications 13 à 22, **caractérisé en ce que** le détergent non ionique est le monododécyléther d'octa-éthylèneglycol (C₁₂E₈) () ou le n-octyloligo-oxyéthylène.

24. Procédé selon l'une quelconque des revendications 15 à 23, **caractérisé en ce que** l'agent de liaison croisée est une molécule organique hétérobifonctionnelle comportant au moins un groupement maléimido et au moins un groupement carboxyle et est de préférence choisi parmi le groupe constitué de dérivés bis-N-succinimidyle et de dérivés succinimidyle photoactivables.

25. Utilisation d'une vésicule selon la revendication 1, pour la fabrication d'une composition destinée à des applications cosmétiques, de diagnostic ou médicales, en particulier destinée à délivrer un médicament ou une substance souhaité à des cellules cibles proliférantes.

26. Utilisation selon la revendication 25, **caractérisée en ce que** lesdites cellules cibles sont incubées in vitro en présence desdites vésicules et **en ce que** lesdites cellules cibles incubées sont facultativement transférables à un organisme in vivo.

27. Utilisation selon la revendication 25 ou 26, **caractérisée en ce que** les cellules cibles sont choisies parmi le groupe constitué de cellules cancéreuses, de cellules leucémiques et de cellules infectées par un virus.

28. Utilisation selon la revendication 27, **caractérisée en ce que** l'activité métabolique ou proliférante desdites cellules cibles est réduite par l'incubation en présence desdites vésicules.

29. Utilisation selon l'une quelconque des revendications 25 à 28, **caractérisée en ce que** ledit médicament ou ladite substance souhaité est un matériau d'acide nucléique qui comprend au moins un oligonucléotide antisens, de préférence un oligonucléotide antisens qui code un ARNm codé en proto-oncogène ou en oncogène.
